# EUROPÄISCHE PATENTSCHRIFT

(11) **EP 1 830 692 B1**
(45) Veröffentlichungstag und Bekanntmachung des Hinweises auf die Patenterteilung: **19.03.2008**
(21) Anmeldenummer: 05818733.7
(22) Anmeldetag: 16.12.2005
(51) Int. Cl.: A61B 1/00, A61B 1/313

(54) **ENDOSKOPARTIGE VORRICHTUNG SOWIE VERFAHREN ZUM UNTERSUCHEN EINES LEBEWESENS**
ENDOSCOPIC DEVICE AND METHOD FOR EXAMINING A LIVING ORGANISM
DISPOSITIF ENDOSCOPIQUE ET PROCEDE POUR ANALYSER UN ETRE VIVANT

(30) Priorität: 31.12.2004 DE 202004020289 U; 21.04.2005 DE 202005006390 U; 01.06.2005 DE 202005008517 U
(43) Veröffentlichungstag der Anmeldung: 12.09.2007
(73) Patentinhaber: Rühland, Dieter, 78224 Singen (DE)
(72) Erfinder: Rühland, Dieter, 78224 Singen (DE)
(74) Vertreter: Nüsse, Stephan
(86) Internationale Anmeldenummer: PCT/EP2005/013562
(87) Internationale Veröffentlichungsnummer: WO 2006/072381

(56) Entgegenhaltungen:
- US-A- 4 076 018
- US-A- 5 605 532
- US-B1- 6 248 060
- US-B1- 6 354 992
- PATENT ABSTRACTS OF JAPAN Bd. 1995, Nr. 10, 30. November 1995 (1995-11-30) & JP 07 184842 A (OLYMPUS OPTICAL CO LTD), 25. Juli 1995 (1995-07-25)
- PATENT ABSTRACTS OF JAPAN Bd. 2003, Nr. 12, 5. Dezember 2003 (2003-12-05) & JP 2003 334157 A (PENTAX CORP), 25. November 2003 (2003-11-25)

## Beschreibung

Die Erfindung betrifft eine endoskopartige Vorrichtung zum Einbringen in einen Kanal eines menschlichen oder tierischen Körpers mit an ein optisches System angeschlossener Röhre engen Durchmessers, die andernends eine Rohrmündung aufweist, wobei in die Röhre ein Heizungssystem integriert ist.

Derartige Vorrichtungen sind als Laparoskope bzw. Thorakoskope bekannt und beispielsweise mit Großbild-Geradeausblick-Optik 0° oder Großbild-Vorausbild-Optik 30° bzw. 45° ausgestattet, wobei bei der Einführung des Mündungsbereiches in den zu untersuchenden Kanal häufig Probleme entstehen. Insbesondere erfolgt häufig ein Beschlagen von am Mündungsbereich installierten optischen Elementen.

US 6,248,060 B1 offenbart eine eingangsgenannte endoskopieartige Vorrichtung, wobei ein Heizungssystem in Form einer elektrischen Heizspule gebildet ist, die in einem Mantelrohr des Endoskops über die Länge des Endoskops eingebettet ist und das gesamte Endoskop auf Körpertemperatur hält.

US 5,605,532 offenbart ein Endoskop mit einem Frontfenster, bei dem in der Nähe des Frontfensters ein thermoelektrisches Heizmittel angeordnet ist, das eine Spule oder einen oder mehrere elektrisch leitende Drähte enthalten kann.

In Kenntnis dieser Gegebenheiten hat sich der Erfinder das Ziel gesetzt, eine Vorrichtung der eingangs erwähnten Art so zu verbessern, dass jene Einführung ohne Schwierigkeiten möglich ist. Vor allem soll das Beschlagen der optischen Elemente unterbunden werden.

Zur Lösung dieser Aufgabe führt die Lehre des unabhängigen Anspruches; die Unteransprüche geben günstige Weiterbildungen an. Zudem fallen in den Rahmen einer Weiterbildung der Erfindung alle Kombinationen aus zumindest zwei der in der Beschreibung, der Zeichnung und/oder den Ansprüchen offenbarten Merkmale. Bei angegebenen Benennungsbereichen sollen auch innerhalb der genannten Grenzen liegende Werte als Grenzwerte offenbart und beliebig einsetzbar sein.

Erfindungsgemäß ist in die Röhre ein Heizungssystem integriert, mit welchem dem Mündungsende bzw. Mündungsbereich der Rohre Wärme zugeführt wird. Dies kann durch ein wärmendes Strömungsmittel erfolgen, nach einem anderen Konzept durch eine elektrische Wärmezuleitung bzw, ein elektrisches Heizelement, das am Mündungsende in der Röhre untergebracht ist. Zudem soll die Temperatur des Heizsystems gesteuert werden können.

Bei der Ausgestaltung der Röhre für das Strömungsmittel sind zum einen etwa achsparallele Führungsräume geringen Querschnitts vorgesehen, die jeweils zur Längsachse der Röhre hin durch einen Teilrohrabschnitt bzw. ein Rinnenprofil begrenzt sind. Oder in der Röhre wird ein Innenrohr angebracht, welches mit der Röhre einen zylindrischen Ringraum als Führungsraum für das Strömungsmittel begrenzt.

Im Rahmen der Erfindung liegt es, dass der Mündungsbereich der Röhre an eine elektrische Wärmezuleitung angeschlossen ist. Nach einem weiteren Merkmal der Erfindung ist in die Röhre ein elektrisches Heizelement eingebracht, deren Mündungsbereich zugeordnet sowie an eine Stromquelle anschließbar gestaltet.

Erfindungsgemäß sind mehrere Heizleiter des Heizelements in einem von zwei Mantelschichten begrenzten Raum unterzubringen und diese bevorzugt parallel zur Mittelachse des Heizelements verlaufen zu lassen.

Eine Mantelschicht enthält bevorzugt ein Glasfasergewebe, das beidseits von einer Deckschicht überspannt ist. Diese aufvulkanisierte/n Deckschicht/en besteht/bestehen vorteilhafterweise aus Siliconwerkstoff, insbesondere aus Silicon RD 6300 E im Falle der innenliegenden Deckschicht sowie Silicon RD 6310 E der äußeren Deckschicht.

Zudem ist vorgesehen, eine Mantelschicht des Heizelements aus Polytetrafluorethylen (PTFE) zu formen.

Bei der Herstellung der Vorrichtung werden die beiden Mantelschichten des Heizelements querschnittlich etwa ringförmig gebogen, und der Raum zwischen ihnen bildet einen Ringraum, der unter Zwischenschaltung der inneren Mantelschicht einen rohrartigen Innenraum des Heizelements umgibt. Als günstig hat es sich erwiesen, auf der Außenfläche des Heizelements einen Heizungsanschluss sowie einen Sensoranschluss anzuordnen, von denen Verbindungskabel ausgehen.

Im Verlauf eines Verfahrens zum Untersuchen eines kanalartigen Bereiches in einem Lebewesen unter Verwendung der endoskopieartigen Vorrichtung mit an ein optisches System angeschlossener Röhre wird dem Mündungsbereich der Röhre Wärme zugeführt und diese, vorzugsweise die Temperatur, vorteilhafterweise gesteuert.

Insgesamt ergibt sich durch die Möglichkeit der Erwärmung des Instrumentes eine erheblich verbesserte Handhabbarkeit, vor allem entfällt die Trübung der optischen Elemente.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in:
- Fig. 1:: eine Seitenansicht eines Laparoskopes bzw. eines Thorakoskopes;
- Fig. 2,3,4:: drei unterschiedlich gestaltete Endabschnitte der Vorrichtung nach Fig. 1;
- Fig. 5:: einen gegenüber der Fig. 5 vergrößerten Ausschnitt einer Vorrichtung im Längsschnitt;
- Fig. 6:: einen Schnitt durch ein Silicontuch;
- Fig. 7, 9:: jeweils eine Draufsicht auf ein Heizelement;
- Fig. 8 bzw. 10:: einen vergrößerten Querschnitt durch das Heizelement der Fig. 7 nach deren Linie VIII-VIII bzw. durch das Heizelement der Fig. 9 nach deren Linie X-X;
- Fig. 11:: eine schematische Skizze zur Zuordnung eines Sensors zu einer Heizung.

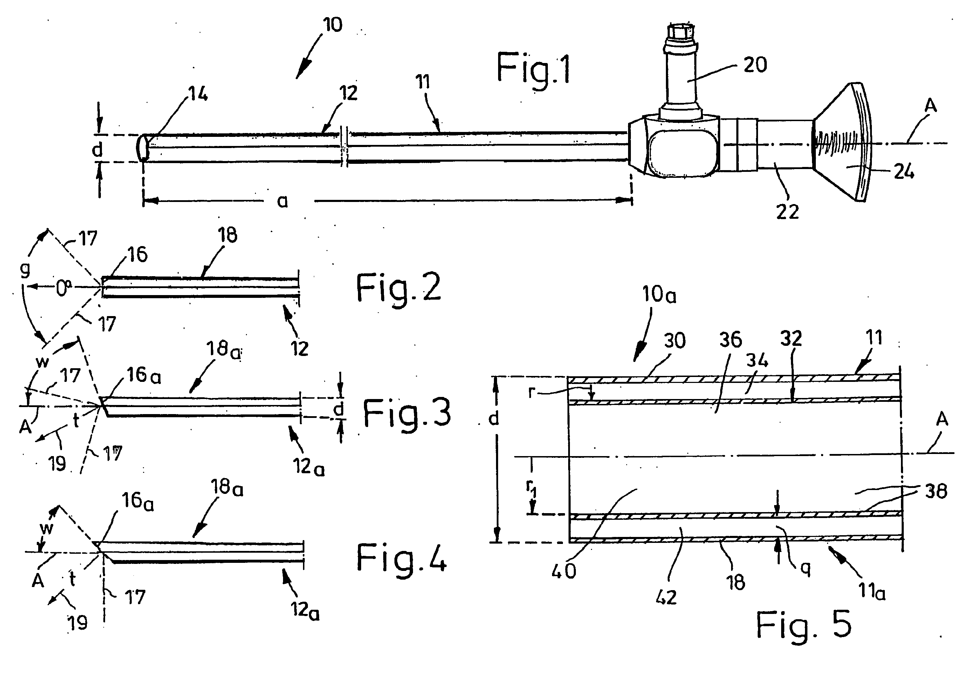

Fig. 1 zeigt ein Laparoskop 10 zur Durchführung von Bauchspiegelungen bzw. ein Thorakoskop für Untersuchungen im Bereich des Brustkorbes, also ein für die Laparoskopie bzw. Thorakoskopie eingerichtetes Endoskop; letzeres ist ein Instrument zum Besichtigen enger Kanäle wie etwa der menschlichen Speiseröhre. Das Laparoskop 10 weist eine in jenen Kanal einzuführende Röhre 12 -- eines Außendurchmessers d von beispielsweise 10 mm sowie einer Länge a von etwa 310 mm -- mit an einem freien Ende vorgesehener Rohrmündung 14 auf, eine andernends angeordnete Beleuchtungseinrichtung 20 sowie ein optisches System 22 von Prismen und Linsen. Bei 24 ist ein zur Rohrlängsachse A koaxialer Ringadapter für ein Auge eines nicht dargestellten Benutzers angedeutet. Dieser Adapter 24 kann auch als Aufnahmeorgan für eine Kamera ausgebildet sein.

In den Fig. 2 bis 4 sind unterschiedlich gestaltete Endabschnitte oder Mündungsbereiche 18, 18ₐ, 18_{b} der Röhre 12, 12ₐ erkennbar. Nicht wiedergegeben ist eine der Rohrmündung 14 bzw. ihrem Mündungsrand 16, 16ₐ zugeordnet sowie den Röhrenquerschnitt schließende transparente Platte.

Der Endabschnitt 18 der Fig. 2 stellt eine autoklavierbare Großbild-Geradeausbild-Optik O° vor mit zur Längsachse A radialem Mündungsrand 16 sowie mit -- nicht erkennbarereingebauter Fiberglas-Lichtleitung. Das zur Längsachse A symmetrische Blickfeld wird von zwei Grenzgeraden 17 bestimmt, diese bestimmen einen Winkel g von 90°.

Endabschnitt 18ₐ der Fig. 3 zeigt eine -- ebenfalls autoklavierbare -- Großbild-Vorausblick-Optik 30° mit einem Anschluss für Fiberglas-Lichtkabel auf einer gegenüberliegenden Seite; ein von einem Sehstrahl 19 mit der Längsachse A gebildeter Winkel t misst hier 30°. In Fig. 4 ist eine Großbild-Optik 45° -- also einem Winkel t von 45° -- dies ebenfalls mit Anschluss für Fiberglas-Lichtkabel auf der gegenüberliegenden Seite. Die Röhren 12ₐ der Fig. 3, 4 enden mit einem zur Längsachse A in einem Winkel w von 70° bzw. 45° geneigten Mündungsrand 16ₐ.

Fig. 5 gibt einen Abschnitt eines Laparoskops 10ₐ mit einer Röhre 11 eines Außendurchmessers d wieder. Nicht erkennbar ist, dass dieses Laparoskop der Länge a -- mit einem zur Längsachse A radialen Mündungsrand 16 -- einen Endabschnitt aufweist, der -- entsprechend Fig. 2 -- als Großbild-Geradeausblick-Optik 0° gestaltet mit eingebauter Fiberglas-Lichtleitung versehen ist.

Der Innenfläche der Rohrwandung 30 der Röhre 11 sind in einer oberhalb der Längsachse A teilweise skizzierten Ausgestaltung -- jeweils von einem Teilrohrabschnitt 32 des Radius r begrenzte -- Rohrräume 34 zugeordnet. Der Querschnitt des Rohrinnenraumes 36 ist somit nicht zylindrisch.

Statt der Teilrohrabschnitte 32 kann -- wie unterhalb der Längsachse A erkennbar -- bei einer anderen Ausgestaltung der Röhre 11ₐ auch ein koaxiales Innenrohr 38 des Innenradius r₁ vorgesehen sein; dieses begrenzt dann einen zylindrischen Innenraum 40 jenes Innenradius r₁ sowie einen äußeren Ringraum 42 einer lichten Weite q der Röhre 11ₐ.

Die beschriebenen Rohrräume 34, 42 dienen der Zuführung von Wärmemitteln an die Rohrmündung 14; ein beheizter Endabschnitt 18, 18ₐ, 18_{b} des Laparaskopes 10ₐ verbessert zum einen die Betrachtungsmöglichkeit für den Benutzer erheblich und bietet zum anderen einen zusätzlichen Schutz für den zu untersuchenden Körper.

Statt der Zuführung von Wärmemitteln wie Heißluft ist auch das elektrische Aufheizen des Mündungsbereiches möglich.

Dieses Prinzip kann auch bei einem Trokar angewendet werden, einer an der Spitze bevorzugt dreikantigen, in einem Röhrchen steckenden Nadel zum Entleeren von Flüssigkeiten aus Körperhöhlen; nach dem Einstecken wird die Nadel herausgezogen und die Flüssigkeit durch das Röhrchen abgeführt.

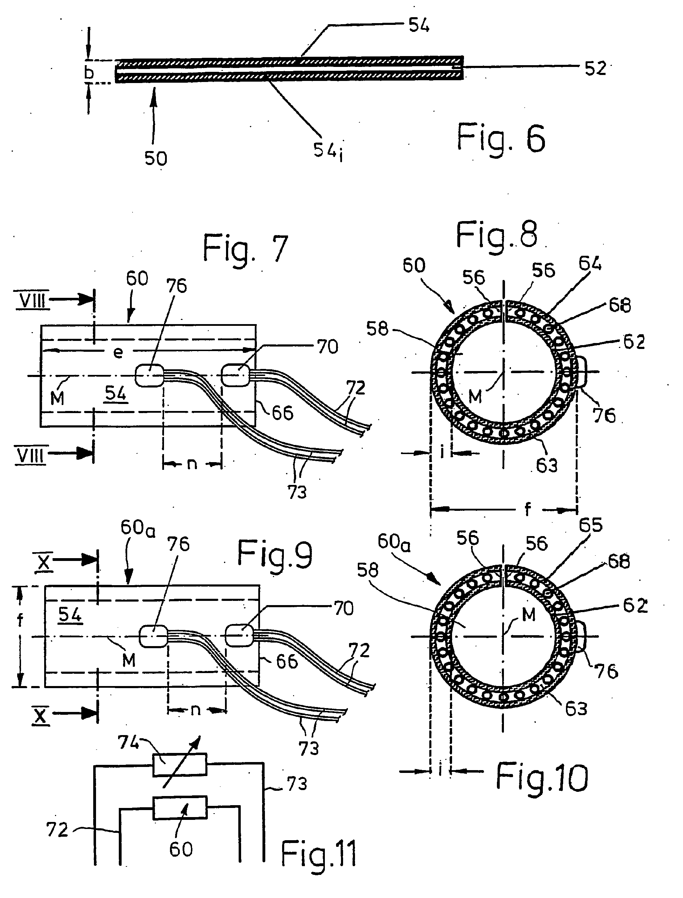

In Fig. 6 ist der Aufbau eines Silicontuches 50 wiedergegeben, das für ein den Fig. 7 bis 10 zu entnehmendes, querschnittlich rohrartiges Heizelement 60 bzw. 60ₐ -- der beispielsweisen Länge e von 60 mm sowie des Außendurchmessers f, der geringfügig kürzer ist als der Innendurchmesser der Röhre 11, 12, 12ₐ -- benötigt wird; letzteres wird in die Röhre 11, 12, 12ₐ eingeführt und dem Mündungsbereich von deren Endabschnitt 18 bzw. 18ₐ .zugeordnet.

Das Silicontuch 50 einer Dicke b zwischen 0,3 mm und 0,5 mm -- hier von 0,3 mm -- weist beidseits eines Glasfasergewebes 52 jeweils eine aufvulkanisierte Deckschicht 54 bzw. 54ₐ aus; die Oberseite 54 besteht aus -- weiter unten erörtertem -- Silicon RD 6310 E, die Unterseite 54ᵢ aus Silicon RD 6300 E. Dieses Silicontuch 50 aus siliconbeschichtetem Glasfasergewebe 52 wird zum einen für die Bildung eines Innenmantels 62 des Heizelementes 60 bzw. 60ₐ eingesetzt, der in einem Abstand i -- von beispielsweise 0,8 mm beim Heizelement 60 sowie von 0, 5 mm beim Heizelement 60ₐ -- in Fig. 7, 8 von einem Außenmantel 64 gleichen Aufbaus umgeben ist. Der Außenmantel 65 des anderen Heizelementes 60ₐ besteht aus Polytetrafluorethylen (PTFE nach DIN 7728T₁).

Im Scheitel der Fig. 8, 10 stehen im übrigen einander die Endkanten 56 des Silicontuches 50 in geringem Abstand gegenüber. In einem vom Innenmantel 62 und Außenmantel 64 bzw. 65 begrenzten Ringraum 63 des Heizelementes 60 bzw. 60ₐ sind -- hier z.B. dreiundzwanzig -- etwa parallel zur Elementmittelachse M verlaufende Heizleiter 68 angeordnet. Diese umgeben -- unter Zwischenschaltung des Innenmantels 62 -- einen rohrartigen Innenraum 58 des Heizelementes 60 bzw. 60ₐ und sind einem Heizungsanschluss 70 zugeordnet, von dem zwei Kabel 72 ausgehen und der nahe einer Schmalkante 66 des Heizelements 60 bzw. 60ₐ an diesem parallel zu jener Elementmittelachse M festgelegt ist. In lichtem Abstand n zum Heizungsanschluss 7o ist ein Sensoranschluss 76 zu erkennen, von dem ebenfalls zwei Kabel 73 ausgehen.

In Fig. 11 ist bei 60 ein Heizelement dargestellt; der Sensor ist mit 74 bezeichnet. In der Zeichnung ist nicht erkennbar, dass die externe Stromversorgung über einen entsprechenden Transformator außerhalb des Endoskopes 10 erfolgt.

Die Temperaturerfassung soll über einen PT 100 nach DIN EN 60751 erfolgen - einmal PT 100 in zwei Leiterschaltungen. Ist das Endoskop 10, 10ₐ mit einer Heizung mit integriertem PT 100 ausgerüstet, gelten die folgenden Abläufe. Der Heizung wird Leistung zugeführt, die Temperatur steigt, der Widerstand des PT 100 verändert sich. Hierzu sei auf die Widerstandstabelle für PT-100 Sensoren gemäß DIN EN 60751 für den Temperaturbereich -200°C ... + 850°C verwiesen. Diese Änderung wird an einer Auswerte-Elektronik -- Regler -- außerhalb des Endoskops 10, 10ₐ ausgewertet. Durch diese Auswertung wird -- im Vergleich zu dem fest eingestellten Temperaturwert -- die Temperatur gesteuert.

Der Regler kann grundsätzlich fest eingestellt sein oder vom Benutzer in engen Grenzen selbst beeinflusst bzw. eingestellt werden. Optional können auch andere Temperaturaufnehmer eingesetzt werden; alle gängigen Thermoelemente -- z.B. Fe Cu Ni Fühler -- funktionieren entsprechend.

Das oben erwähnte Silicon RD 6300 E ist eine lösungsmittelhaltige Siliconkautschukdispersion für Gewebebeschichtung, peroxidvernetzt. Die Vulkanisation erfolgt nach vorhergehender Verdunstung des Lösemittels, beispielsweise 10 min bei 135°C oder 5 min bei 180°C. Die Viskosität beträgt 30 000 bis 80 000 m Pa s.

Der folgenden Tabelle sind dazu Eigenschaften in Richtwerten zu entnehmen.

| ungetempert getempert* | | | |
|---|---|---|---|
| Härte (DIN 53 505) | Shore A | 83 | 81 |
| Dichte (DIN 53 479 A) | g/cm³ | 1.60 | 1.60 |
| Reißfestigkeit (DIN 53 504-S1) | N/mm² | 3.3 | 4.9 |
| Reißdehnung (DIN 53 504-S1) | % | 70 | 70. |
| Weiterreißwiderstand (ASTM D 642 B) | N/mm | 8.6 | 10.0 |
| Rückprallelastizität (DIN 53 512) | % | 58 | 53 |
| Druckverformungsrest (DIN 53 517 22 h/175°C) | % | 104 | 100 |
| Wärmeleitfähigkeit | % | 0.74 | 0.75 |
| Sauerstoffindex LOI (ASTM D 2863-70) | | 29 | 21 |
| Durchschlagsfestigkeit | VDE 0303 | kV/mm | 20 |
| Spezifischer Durchgangswiderstand | VDE 0303 | Ω·cm | 6,9·10⁴ |

| | | | |
|---|---|---|---|
| *4h 200°C | | | |

Die Eigenschaften des Glasfasergewebes 52 zeigen die folgenden Tabellen auf.
(a) Einseitig -- mit RD 6310 E -- beschichtetes Glasfasergewebe 4000-21:

| | | |
|---|---|---|
| Beschichtungsdicke | 0,3 mm | |
| Beschichtungsart | Roh mit Folie abgedeckt | |
| Beschichtungsmenge | 415 gr/m² | |
| Temperaturbeständig | -60°C - 250°C | |
| Trennfolie | 11-02-007 | PE-Flachfolie |
| Lösungsmittel | Vor Folienaufbringung abgedampft | |
| Folienaufbringung | Ohne Falten und Blasen | |
| Wärmedurchgang (k-Faktor) | 0,74 w/mk | |
| Hochspannungsfestigkeit | | |
| Durchschlagsfestigkeit | 20kv/mm | Richtwert (VDE 0303) |
| Spezifischer Durchgangswiderstand | 6,9*10^14 Ohm*cm | Richtwert (VDE 0303) |

b) einseitig beschichtetes Glasfasergewebe 4000-19:

| | | |
|---|---|---|
| Beschichtungsdicke | 0,2mm | |
| Beschichtungsart | Vulkanisiert | |
| Beschichtungsmenge | 160 gr/m² | |
| Temperaturbeständig | -60°C - 250°C | |
| Durchdringung | keine | auf der nicht beschichteten Seite |
| Wärmedurchgang (k-Faktor) | 0,25 w/mk | |

## Patentansprüche

1. Endoskopartige Vorrichtung zum Einbringen in einen Kanal eines menschlichen oder tierischen Körpers mit an ein optisches System (20) angeschlossener Röhre (11, 11ₐ, 12, 12ₐ) engen Durchmessers (d), die andernends eine Rohrmündung (14) aufweist, wobei in die Röhre (11, 11ₐ, 12, 12ₐ) ein Heizungssystem integriert ist,
**dadurch gekennzeichnet,**
**dass** in die Röhre (11, 11ₐ, 12ₐ) ein mit einer Innenmantelschicht (62) und einer Außenmantelschicht (64,65) gebildetes querschnittlich rohrartiges elektrisches Heizelement (60, 60ₐ) eingebracht ist, wobei die Innenmantelschicht (62) ein Glasfasergewebe (52) enthält, das beidseits von einer Deckschicht (54, 54ᵢ) überspannt ist, und wobei das Heizelement (60, 60ₐ) dem Mündungsbereich (18, 18ₐ) der Rohre (11, 11ₐ, 12, 12ₐ) zugeordnet sowie an eine Stromquelle anschließbar ausgebildet ist, wobei mehrere Heizleiter (68) des Heizelements (60, 60ₐ) in einem von der Innenmantelschicht (62), und der Außenmantelschicht (64/65) des Heizelements (60, 60ₐ) begrenzten Raum (63) verlaufen, mit welchem Heizungssystem dem Mündungsbereich (18, 18ₐ, 18_{b}) der Röhre Wärme zuführbar ist.

2. Vorrichtung nach Anspruch 1, **dadurch gekennzeichnet, dass** die Temperatur des Heizungssystems steuerbar ist.

3. Vorrichtung nach Anspruch 1 oder 2, **gekennzeichnet durch** zumindest einen in die Röhre (11, 11ₐ) integrierten Führungsraum (34, 42) für ein wärmendes Strömungsmittel.

4. Vorrichtung nach Anspruch 3, **gekennzeichnet durch** einen an die Innenfläche der Rohrwandung (30) der Röhre (11) angefügten Führungsraum (34) geringen Querschnitts.

5. Vorrichtung nach Anspruch 4, **dadurch gekennzeichnet, dass** der Führungsraum (34) zur Längsachse (A) der Röhre (11) hin durch einen Teilrohrabschnitt (32) bzw. ein Rinnenprofil begrenzt ist.

6. Vorrichtung nach Anspruch 3, **gekennzeichnet durch** ein von einem Innenrohr (38) begrenzten Ringraum (42) als Führungsraum der Röhre (11ₐ), welcher den Innenraum (40) des Innenrohres umgibt.

7. Vorrichtung nach Anspruch 1, **dadurch gekennzeichnet, dass** der Mündungsbereich (18, 18ₐ) der Röhre (11, 11ₐ, 12ₐ) an eine elektrische Wärmezuleitung angeschlossen ist.

8. Vorrichtung nach Anspruch 1, **dadurch gekennzeichnet, dass** die Heizleiter (68) etwa parallel zur Mittelachse (M) des Heizelements (60, 60ₐ) verlaufen.

9. Vorrichtung nach Anspruch 1, **dadurch gekennzeichnet, dass** die aufvulkanisierte/n Deckschicht/en (54, 54ᵢ) aus Siliconwerkstoff besteht/bestehen.

10. Vorrichtung nach Anspruch 9, **gekennzeichnet durch** eine Deckschicht (54) aus Silicon RD 6310 E.

11. Vorrichtung nach Anspruch 9, **gekennzeichnet durch** eine Deckschicht (54ᵢ) aus Silicon RD 6300 E.

12. Vorrichtung nach Anspruch 1 und 10, **dadurch gekennzeichnet, dass** die innere Deckschicht (54ᵢ) aus Silicon RD 6300 E besteht.

13. Vorrichtung nach Anspruch 1, **dadurch gekennzeichnet, dass** eine Mantelschicht (65) des Heizelements (60ₐ) aus Polytetrafluorethylen (PTFE) geformt ist.

14. Vorrichtung nach einem der Ansprüche 1 bis 13, **dadurch gekennzeichnet, dass** die beiden Mantelschichten (62, 64/65) des Heizelements (60, 60ₐ) querschnittlich etwa ringförmig gebogen sind und der Raum zwischen ihnen einen Ringraum (63) bildet, der unter Zwischenschaltung der inneren Mantelschicht (62) einen rohrartigen Innenraum (58) des Heizelements umgibt.

15. Vorrichtung nach einem der Ansprüche 1 bis 14, **dadurch gekennzeichnet, dass** auf der Außenfläche des Heizelements (60, 60ₐ) ein Heizungsanschluss (70) sowie ein Sensoranschluss (76) angeordnet sind, von denen Verbindungskabel (72 bzw. 73) ausgehen.

## Claims

1. Endoscope-like device for insertion in a canal of a human or animal body, having a tube (11, 11ₐ, 12, 12ₐ) of narrow diameter (d) which is connected to an optical system (20) and which has a tube orifice (14) at the other end, a heating system being integrated in the tube (11, 11ₐ , 12, 12ₐ),
**characterised in that**
into the tube (11, 11ₐ, 12ₐ) is introduced a cross-sectionally tubular electric heating element (60, 60ₐ) formed with an inner sheath layer (62) and an outer sheath layer (64, 65), the inner sheath layer (62) containing a glass fibre cloth (52) which is spanned on both sides by a covering layer (54, 54ᵢ), and the heating element (60, 60ₐ) being associated with the orifice region (18, 18ₐ) of the tube (11, 11ₐ, 12, 12ₐ) and connectable to a current source, several heating conductors (68) of the heating element (60, 60ₐ) running in a space (63) which is defined by the inner sheath layer (62) and the outer sheath layer (64/65) of the heating element (60, 60ₐ), with which heating system heat can be delivered to the orifice region (18, 18ₐ, 18_{b}) of the tube.

2. Device according to claim 1, **characterised in that** the temperature of the heating system is controllable.

3. Device according to claim 1 or 2, **characterised by** at least one guide chamber (34, 42) which is integrated in the tube (11, 11ₐ), for a heating flow medium.

4. Device according to claim 3, **characterised by** a guide chamber (34) of small cross-section joined to the inner surface of the tube wall (30) of the tube (11) .

5. Device according to claim 4, **characterised in that** the guide chamber (34) is delimited from the longitudinal axis (A) of the tube (11) by a partial tube section (32) or a groove profile.

6. Device according to claim 3, **characterised by** an annular chamber (42) defined by an inner tube (38) as the guide chamber of the tube (11ₐ), which surrounds the interior (40) of the inner tube.

7. Device according to claim 1, **characterised in that** the orifice region (18, 18ₐ) of the tube (11, 11ₐ, 12ₐ) is connected to an electrical heat supply wire.

8. Device according to claim 1, **characterised in that** the heating conductors (68) run approximately parallel to the centre axis (M) of the heating element (60, 60ₐ).

9. Device according to claim 1, **characterised in that** the covering layer(s) (54, 54ᵢ) which is/are applied by vulcanisation is/are made of silicone material.

10. Device according to claim 9, **characterised by** a covering layer (54) made of RD 6310 E silicone.

11. Device according to claim 9, **characterised by** a covering layer (54ᵢ) made of RD 6300 E silicone.

12. Device according to claims 1 and 10, **characterised in that** the inner covering layer (54ᵢ) is made of RD 6300 E silicone.

13. Device according to claim 1, **characterised in that** a sheath layer (65) of the heating element (60ₐ) is formed from polytetrafluoroethylene (PTFE).

14. Device according to any of claims 1 to 13, **characterised in that** the two sheath layers (62, 64/65) of the heating element (60, 60ₐ) are cross-sectionally curved for example in a ring shape, and the space between them forms an annular chamber (63) which, with the interposition of the inner sheath layer (62), surrounds a tubular inner chamber (58) of the heating element.

15. Device according to any of claims 1 to 14, **characterised in that** on the outer surface of the heating element (60, 60ₐ) are arranged a heating connection (70) and a sensor connection (76) from which connecting cables (72 or 73) extend.

## Revendications

1. Dispositif de type endoscopique destiné à être introduit dans un canal d'un corps humain ou animal, avec des tubes (11, 11ₐ, 12, 12ₐ) de diamètre réduit (d) reliés à un système optique (20), lesquels présentent un orifice de tube (14) à leur autre extrémité, un système de chauffage étant intégré aux tubes (11, 11ₐ, 12, 12ₐ),
**caractérisé**
**en ce qu'**un élément de chauffage électrique (60, 60ₐ) de section transversale tubulaire formé avec une couche d'enveloppe intérieure (62) et une couche d'enveloppe extérieure (64, 65) est inséré dans les tubes (11, 11ₐ, 12, 12ₐ), la couche d'enveloppe intérieure (62) comprenant un tissu de fibres de verre (52) recouvert d'une couche de couverture (54, 54ᵢ) sur ses deux côtés, l'élément de chauffage (60, 60ₐ) étant associé à la zone d'orifice (18, 18ₐ) des tubes (11, 11ₐ, 12, 12ₐ) et prévu pour pouvoir être raccordé à une source de courant, plusieurs résistances chauffantes (68) de l'élément de chauffage (60, 60ₐ) s'étendant dans un espace (63) délimité par la couche d'enveloppe intérieure (62) et la couche d'enveloppe extérieure (64/65) de l'élément de chauffage (60, 60ₐ), la chaleur pouvant être conduite vers la zone d'orifice (18, 18ₐ, 18_{b}) des tubes grâce à ce système de chauffage.

2. Dispositif selon la revendication 1, **caractérisé en ce que** la température du système de chauffage peut être commandée.

3. Dispositif selon la revendication 1 ou 2, **caractérisé par** au moins une chambre de guidage (34, 42) d'un fluide chauffant, intégrée dans les tubes (11, 11ₐ).

4. Dispositif selon la revendication 3, **caractérisé par** une chambre de guidage (34) de section transversale réduite accolée à la surface intérieure de la paroi (30) des tubes (11).

5. Dispositif selon la revendication 4, **caractérisé en ce que** la chambre de guidage (34) est limitée par un tronçon de tube (32) ou par un profilé en gouttière selon l'axe longitudinal (A) des tubes (11).

6. Dispositif selon la revendication 3, **caractérisé par** une chambre annulaire (42) limitée par un tube intérieur (38), en tant que chambre de guidage des tubes (11ₐ) entourant l'espace intérieur (40) du tube intérieur.

7. Dispositif selon la revendication 1, **caractérisé en ce que** la zone d'orifice (18, 18ₐ) des tubes (11, 11ₐ, 12ₐ) est raccordée à une conduite de chauffage électrique.

8. Dispositif selon la revendication 1, **caractérisé en ce que** les conduites de chauffage (68) s'étendent sensiblement parallèlement à l'axe central (M) de l'élément de chauffage (60, 60ₐ).

9. Dispositif selon la revendication 1, **caractérisé en ce que** la (les) couche(s) de couverture (54, 54ᵢ) vulcanisée(s) est (sont) réalisée(s) en matériau silicone.

10. Dispositif selon la revendication 9, **caractérisé par** une couche de couverture (54) en silicone RD 6310 E.

11. Dispositif selon la revendication 9, **caractérisé par** une couche de couverture (54ᵢ) en silicone RD 6300 E.

12. Dispositif selon les revendications 1 et 10, **caractérisé en ce que** la couche de couverture intérieure (54ᵢ) est réalisée en silicone RD 6300 E.

13. Dispositif selon la revendication 1, **caractérisé en ce qu'**une couche d'enveloppe (65) de l'élément de chauffage (60ₐ) est moulée en polytétrafluoroéthylène (PTFE).

14. Dispositif selon l'une des revendications 1 à 13, **caractérisé en ce que** les deux couches d'enveloppe (62, 64/65) de l'élément de chauffage (60, 60ₐ) sont cintrées en section transversale sensiblement en forme annulaire, et **en ce que** l'espace entre elles forme une chambre annulaire (63) qui, par interposition de la couche d'enveloppe intérieure (62), entoure une chambre intérieure tubulaire (58) de l'élément de chauffage.

15. Dispositif selon l'une des revendications 1 à 14, **caractérisé en ce qu'**un raccord de chauffage (70) ainsi qu'un raccord de capteur (76) sont disposés sur la surface extérieure de l'élément de chauffage (60, 60ₐ), des câbles de connexion (72 et 73) partant desdits raccords.
